# NEW EUROPEAN PATENT SPECIFICATION

(11) **EP 0 831 901 B2**
(45) Date of publication and mention of the opposition decision: **07.12.2005**
(45) Mention of the grant of the patent: 19.09.2001
(21) Application number: 96920790.1
(22) Date of filing: 04.06.1996
(51) Int. Cl.: A61K 39/385, A61K 39/00, A61K 39/39

(54) **VACCINE COMPRISING A POLYSACCHARIDE ANTIGEN-CARRIER PROTEIN CONJUGATE AND FREE CARRIER PROTEIN**
VAKZINE MIT EINEM POLYSACCHARIDE ANTIGEN-TRÄGERPROTEIN KONJUGAT UND FREIEN TRÄGERPROTEIN
VACCINS COMPRENANT UN CONJUGUE ANTIGENE DE POLYSACCHARIDE-PROTEINE PORTEUSE ET UNE PROTEINE PORTEUSE LIBRE

(30) Priority: 07.06.1995 US 472639; 23.06.1995 GB 9512827; 01.07.1995 GB 9513443; 15.12.1995 GB 9525657
(43) Date of publication of application: 01.04.1998
(60) Divisional application: 00203772.9
(73) Proprietor: SmithKline Beecham Biologicals s.a., 1330 Rixensart (BE)
(72) Inventor: SLAOUI, Moncef Mohamed, Rue de l'Institut 89 1330 Rixensart (BE); HAUSER, Pierre, Rue de l'Institut 89 1330 Rixensart (BE)
(74) Representative: Dalton, Marcus Jonathan William
(86) International application number: PCT/EP1996/002436
(87) International publication number: WO 1996/040242

(56) References cited:
- EP-A- 0 208 375
- EP-A- 0 594 950
- US-A- 4 644 059
- INFECTION AND IMMUNITY, vol. 62, 1994, pages 9-14, XP000602292 BARINGTON, T. ET AL.: "Opposite effects of actively and passively acquired immunity to the carrier on responses of human infants to a Haemophilus influenzae type b conjugate vaccine"
- BIOLOGICALS, vol. 22, 1994, pages 353-360, XP000603003 CORBEL, M.J.: "Control testing of combined vaccines: a consideration of potential problems and approches"
- BULLETIN DE L'ACAD MIE NATIONALE DE M DECINE, vol. 177, 1993, pages 1381-1387, XP000602905 B GU , P.: "Vaccins haemophilus influenzae"
- INFECTION AND IMMUNITY, vol. 61, 1993, pages 432-438, XP000602293 BARINGTON, T. ET AL.: "Non-epitope-specific suppression of the antibody response to Haemophilus influenzae type b conjugate vaccines by preimmunization with vaccine components"
- CLINICAL IMMUNOTHERAPEUTICS, vol. 4, November 1995, pages 376-386, XP000602898 FRASCH, C.E.: "Haemophilus influentae type b conjugate and combination vaccines"
- Scheifele et al., Can Med Assoc J., vol 149, pp. 1105-1112, 1993
- Kaplan et al., J. Pediatr., vol 124, 323, 1994

## Description

The present invention relates to combination vaccines comprising a conjugated polysaccharide antigen linked to a carrier protein, and wherein the carrier protein is also present as a free antigen in the vaccine composition. In particular the vaccine composition of the present invention relates to a multivalent vaccine, that is a vaccine for the amelioration or treatment of more than one disease states. The present invention also relates to the production and use of such a vaccine in medicine.

Vaccines that utllise polysaccharides are known in the art. For example a vaccine for the prevention of Haemophilus influenzae b (Hib) infections are based on the capsular Polysaccharide (PRP) conjugated with a carrier protein. Typically the carrier protein is a diphtheria or tetanus toxoid. It has also been suggested to produce a vaccine for the prevention of Streptococcus pneumonia which is based on capsular polysaccharide conjugated to a carrier protein such as tetanus toxoid or diphtheria toxoid. Examples of such conjugate vaccine antigens are disclosed in US 4 365 170 US 4 673 574 EP 208 375 EP 477508 and EP 161 188.

It is desirable to administer such conjugate va nes with other antigens or vaccines at the same time and this can involve multiple injections. Problems associated with multiple injections include a more complicated administration procedure and a large total injection volume. This is a particularly acute problem when the vaccine is intended for infants.

It has therefore been proposed to produce combination vaccines. One well known combination vaccine provides protection against Diphtheria, tetanus and B. pertusis infections. This vaccine comprises a pertussis component (either killed whole cell B. pertussis or accellular pertuss is which typically consists of up to three antigens - PT, FHA and 69kDa) and toxoided diphtheria and tetanus toxin. Such vaccines are often referred to as DTPw (whole cell) or DTPa (accellular). Bégué, P. (Bull Acad Natle Med, 177:1381-1390 (1993)) reviews the efficacy of existing Hib conjugate vaccines. This reference also discusses DTP-Hib vaccines where the Hib is coadministered (or admixed just prior to use) with the DTP component. Corbel, M. (Biologicals, 22:353-360 (1994)) discusses problems associated with combination vaccines. This reference further notes that DTP-HIb vaccines may produce different results than DTP and HIb components administered separately. Barington et al. (infect immun, 61:432-438 (1993) and (Infect Immun 62:9-14 (1994)) discuss the effects of antibody response suppression (interference) of Hib conjugate vaccines when individuals have been preimmunized with one of the individual components of the conjugate vaccine. EP 594 950 discloses a combination vaccine containing DTP and Hib fragments (oligosaccharide) conjugated to CRM₁₉₇. Kovel et. al. (The Journal of Pediatrics 120: 84-87, 1992) discloses an acellular diphtheria-tetanus-pertussis and Haemophilus-diphtheria toxoid conjugate vaccine given in combination. Scheifele et al. (Can. Med. Assoc. J. 1993, 149: 1105-1112) discloses a OTP_{w} PRP-tetanus toxoid combination vaccine as does Ferreccio et al. (Pediatr. Infect. Dis J. 1991, 10: 764-771) and Siber et al. (Vaccine, 1995, 13: 525-531).

It would be desirable to add polysaccharide conjugate vaccines to such a combination. However we have found that simple mixing of the components results in a reduction of antibody titres to the polysaccharide component and that the persistence of protective levels of such antibody titres is reduced. Thus the immunogenicity of conjugate PRP Hlb polysaccharide, to detoxified Tetanus Toxoid (TT) has been observed to decrease in infants immunised with TT-PRP conjugate combined to DTPa as compared to infants immunised with TT-PRP conjugate and DTPa concomitantly, but at separate sites.

in the monovalent situation, ie when a vaccine comprises just a single polysaccharide (PS) conjugated to a carrier protein, the higher the protein content of the conjugate the higher the antibody response to the polysaccharide. This is thought to be because induction of a T cell dependent polysaccharide specific antibody response requires the covalent coupling of a protein carrier to the polysaccharide. The protein carrier needs to be present at concentrations (ie PS to protein ratio) such that a good TH cell response to the carrier is induced, and that this TH response can recognise PS specific B cell clones expressing peptides derived from the covalently coupled carrier protein.

Surprisingly and in complete contrast to the position in the monovalent vaccine, the present inventors have discovered that when the polysaccharide conjugate component is part of a multivalent vaccine comprising a free form of the carrier protein tetanus toxoid, the immunogenicity of the polysaccharide component is improved by reducing the protein content of the conjugate antigen.

Accordingly the present invention provides a combined vaccine comprising a polysaccharide antigen derived from Haemophilus influenzae type B, streptococcus pneumonide, or Neisseria meningitidis A, B or C, conjugated to a carrier protein and a OTPa vaccine comprising diphtheria toxoid, tetanus toxoid and an acellular pertussis component characterised in that the ratio of polysaccharide to carrier protein in the conjugate is from 1:0.3 to 1:2 (w/w) and in that the carrier protein is tetanus toxoid which is also present as free antigen in the DTPa vaccine. A preferred ratio is 1:0.5 to 1:1.5, typically in the order of 1:1.

The polysaccharide conjugate may be prepared by any known coupling technique. For example the polysaccharide can be coupled via a thioether linkage. This conjugation method relies on activation of the polysaccharide with 1-cyano-4-dimethylamino pyridinium tetrafluoroborate (CDAP) to form a cyanate ester. The activated polysaccharide may thus be coupled directly or via a spacer group to an amino group on the carrier protein. Preferably, the cyanate ester is coupled with hexane diamine and the amino-derivatised polysaccharide is conjugated to the carrier protein using heterofigation chemistry involving the formation of the thioether linkage. Such conjugates are described in PCT published application WO93/15760 Uniformed Services University.

The conjugates can also be prepared by direct reductive amination methods as described in US 4365170 (Jennings) and US 4673574 (Anderson). Other methods are described in EP-0-161-188, EP-208375 and EP-0-477508.

A further method involves the coupling of a cyanogen bromide activated polysaccharide derivatised with adipic acid hydrazide (ADH) to the protein carrier by Carbodilmide condensation (Chu C. et al Infect Immunity, 1983 245 256).

Preferably the compositions of the invention contain a suitable adjuvant.

A preferred adjuvant is MPL (3-deacylated monophosphoryl lipid A, also known as 3D-MPL). 3D-MPL is known from GB 2 220 211 (RibI) as a mixture of 3 De-O-acylated monophosphoryl lipid A with 4, 5 or 6 acylated chains and is manufactured by Ribi Immunochem, Montana. A preferred form of MPL is disclosed in International Patent Application 92/116556.

Another adjuvant which may be used In the present invention is known as QS21. QS21 is a Hplc purified non toxic fraction of a saponin from the bark of the South American tree Quillaja saponaria molina and its method of its production is disclosed (as QA21) in US patent No. 5,057,540.

Combinations of QS21 and 3 D-MPL are known to produce a synergistically effective vaccine formulation and are described in International Patent application WO 94/00153.

Often the Hib component vaccines of the invention will not require any specific carrier and be formulated in an aqueous or other pharmaceutically acceptable buffer. In some cases it may be that the entire vaccine of the present invention will be presented in an oil in water emulsion, or other suitable vehicle, such as for example, liposomes, microspheres or encapsulated antigen particles.

The vaccine formulations may contain further antigens. Antigen or antigenic compositions known in the art can be used in the compositions of the invention, including antigen or antigenic compositions derived from HIV-1, (such as gp120 or gp160), human or animal herpes viruses, such as gD or derivatives thereof or immediate Early protein such as ICP27 from HSV1 or HSV2, cytomegalovirus (especially human)(such as gB or derivatives thereof), Varicella Zoster Virus (such as gpl, II or III), or from a hepatitis virus such as hepatitis B virus for example Hepatitis B Surface antigen or a derivative thereof, hepatitis A virus, hepatitis C virus and hepatitis E virus, or from other viral pathogens, such as Respiratory Syncytial virus, human papilloma virus or Influenza virus, or polio virus such as Inactivated Polio Virus (IPV) or derived from bacterial pathogens such as, Salmonella, Neisseria, particularly Neisseria Meningitis A, B, or C. Borreila (for example OspA or OspB or derivatives thereof), or Chlamydia, or derived from parasites such as plasmodium or Toxoplasma.

Particularly preferred vaccines of the invention include, DTPa Hib; and DTPa Hib Hep B; and DTPa Hib Hep B IPV.

It will be appreciated that in the combination vaccine of the invention the Hib component may be formulated with the other antigens just prior to administration. Thus a lyopholised Hib component may be reconstituted prior to use, by mixing with the aqueous formulation of other antigens.

The above combinations may optionally include a component which is protective against Hepatitis A.

Suitable components for use in such vaccines are already commercially available and details may be obtained from the World Health Organisation. For example the IPV component may be the Salk inactivated polio vaccine. The Diphtheria, Tetanus and Pertussis vaccine may comprise an accellular product such as infanrix® DTPa (SmithKline Beecham Biologicals) . The component affording protection against Hepatitis A is preferably the product known as 'Havrix'® (SmithKline Beecham Biologicals) which is a killed attenuated vaccine derived from the HM-175 strain of HAV [see 'Inactivated Candidate Vaccines for Hepatitis A' by F.E. Andre, A Hepburn and E.D'Hondt, Prog Med. Virol. Vol 37, pages 72-95 (1990) and the product monograph 'Havrix'® published by SmithKline Beecham Biologicals (1991)]. The Hepatitis B component may comprise the 'S' antigen as in 'Engerix-B'®.

Advantageously the combination vaccine according to the invention is a paediatric vaccine.

Vaccine preparation is generally described in Vaccine Design - the subunit and Adjuvant approach edited by Michael F Powell and Mark Newman, Plenum Press. Encapsulation within liposomes is described, for example, by Fullerton, US Patent 4,235,877. Conjugation of proteins to macromolecules is disclosed, for example, by Likhite, US Patent 4,372,945 and by Armor et al., US Patent 4,474,757.

The amount of antigen in each vaccine dose is selected as an amount which induces an immunoprotective response without significant, adverse side effects in typical vaccinees. Such amount will vary depending on which specific immunogens are employed. Generally it is expected that each dose will comprise 1-1000ug of total immunogen, preferably 2-100ug, most preferably 4-40ug. An optimal amount for a particular vaccine can be ascertained by standard studies involving observation of antibody titres and other responses in subjects. Following an initial vaccination, subjects may receive one or two booster injections at about 4 weeks intervals.

The following examples will illustrate the invention.

### Examples

### Example 1a) Vaccine comprising DTPa - Hib PRP-TT - and HBsAg.

### Production of Hib TT conjugate

### Materials and Methods

### Materials

The Hib PRP is extracted and purified from inactivated cell cultures. The purified material meets the WHO and US specifications in terms of residual protein, nucleic acid, endotoxin, structural sugars and molecular size distribution.

Tetanus toxoid produced by Behringwerke meets also the WHO specifications. The material is further purified by acid pH precipitation and gel filtration chromatography to isolate the monomeric form (150 kDa) of TT.

### Activation and Coupling Chemistry

20 mg of Hib PRP were dissolved in 4 ml of 2 M Na Cl.

150 µl of CDAP (1-cyano-4-dimethylamino-pyridinium tetrafluoroborate) was added to the polysaccharide solution (from a 100 mg/ml stock solution in acetronitrile). One minute later, 300 µl of triethylamine 0.2 M was added. The activation of the polysaccharide was performed during 2 minutes at 0°C at pH 9.5-10.

20 mg or 40 mg of the Tetanus toxoid (initial PRP/Protein ratio of 1/1 or 1/2) at a concentration of 15 mg/ml was added and the coupling reaction was performed at 25°C for 1 hour. Then the reaction was quenched for 1 hour at 25°C with 3 ml of 1 M glycine solution pH 5.0 and then overnight at 4°C.

The conjugate is purified by gel filtration chromatography using a Sephacryl HR 500 gel filtration column equilibrated with 0.2 M NaCl. The carbohydrate and protein content of the eluted fractions was determined. The conjugate was pooled in and sterile filtered. The PRP/protein ratio and free PS content in the conjugate preparation were determined.

### Example 1b)

### DTP Hib PRP TT HBsAg Combination Vaccine Formulation

Formulation of DTPa (comprising Diphtheria toxoid, Tetanus toxoid, pertussis toxoid, pertactin and FHA) with or without Hepatitis B vaccines are known in the art. For example such a vaccine is disclosed in International Patent application No WO 93/24148. The Conjugate as described in example 1 was added to the vaccine and mixed prior to injection for immunogenicity studies.

In the present studies the following steps were undertaken:
- adjustment of phenoxy-ethanol (bacteriostatic) content to 5 mg/ml
- adjustment of pH at 6.1 ± 0.1
- adjustment of PRP concentration up to 100 µg/ml
- addition of DTPaHBV (0.5 ml) to 0.1 ml of the PRP-CDAP-TT solution.

The final composition of the vaccine was for one dose:
- PRP'P 10 µg
- D 25 Lf
- T 10 Lf
- PT 25 µg
- FHA 25 µg
- 69K 8 µg
- Hep B 10 µg
- A1 (OH)₃ Behring 0.35 mg
- A1 (OH)₃ Superpos 0.15 mg
- AlPO₄ Superpos 0.20 mg
- NaCl 150 mM
- 2ϕEt-OH 3 mg (→5 mg/ml)
- pH 6.1 ± 0.1
- Vol 0.6 ml

### Example 2

### Immunogenicity

The immunogenicity of Haemophilus Influenzae type B derived polysaccharide:protein (weight:weight) ratios (1:3, 1:2 and 1:1), using two different coupling chemistries, is compared after a first and second inoculation in young OFA rats.

The data obtained at 28 days after the first vaccine inoculation clearly show an improved anti PS antibody titre correlating with a decreased protein content in the conjugate vaccine, when the conjugate antigen is combined to DTPa Hepatitis B, but not when the conjugate is injected alone. This is in clear contrast to the situation observed when the conjugate vaccine is given alone, where the Lower PS-TT ratio (ie more carrier protein) results in a better antibody response to the Polysaccharide (see day 42 anti PS titre of Groups 1 to 3 going from 25, to 18 to 5 µg/ml with a decreasing amount of TT, while they go from 3.4 to 8 to 19 µg/ml for the same TT contents, but when given in combination with DTPa Hep B vaccine.

### Example 3

### Effect of TT/PS ratio on compatibility of Hib with DTPa

Groups of 10 rats (OFA, 5 weeks of age, female) were immunised 2 times at 24 days interval by the subcutaneous route with the different TT-PRP vaccines given at 2 dosages: 0.5µg PS/rat (table 2) or 0.05µg PS/rat (table 3). The Hib001 vaccine was reconstituted with saline or 1 dose of DTPa (lot 119) to have a concentration of 10µg PS/ml. The liquid vaccines were diluted with saline or 1 human dose of DTPa to have 10µg PS/ml. The vaccines were then further diluted in saline to have 2.5µg PS/ml or 0.25µg PS/ml. The rats were injected with 200 µl of each vaccine within one hour after dilution.

The animals were bled at day 24 and day 38 and the anti-PRP 1 gG Abs were measured by ELISA in individual sera and expressed in γ/ml. The GMT was calculated for each group.

### Example 4

### Comparison of Immunogenicity of DTPa, and DTPa Hepatitis B vaccine mixed with low ratio or 'normal' PRP:TT

Groups of 10 rats (OFA, 1 week of age) were immunised three times (SC) at two week intervals with 0.5 µg of PS combined 1 hour before injection with 1/20th a human dose of DTPa or DTPa HepB. The animals were bled 2 weeks after the third dose and the antibodies against pertussis toxoid (PT), FHA, pertactin (69k), Diphtheria toxoid (D) Tetanus toxoid (T) and Hepatitis B surface antigen (HBS) measured by Elisa. The titres, expressed as a mid point dilution using a reference are shown in Table 4.

The results of this experiment together with the result from example 3 demonstrate that the Hib response is enhanced in a combination vaccine, if the amount of protein in the conjugate is reduced. Moreover, there is no significant interference to the immune response of any other component of the vaccine.

**TABLE 1**

| **Conjugate** | **Ps/Protein Ratio Weight:Weight** | **Conjugate alone** | | **Conjugate plus Hep B and DTPa** | |
|---|---|---|---|---|---|
| | | **D28 (PI)** | **D42 (PII)** | **D28 (PI)** | **D42 (PII)** |
| Buffer | | 0.05 | 0.11 | 0.05 | 0.11 |
| | | | | | |
| PRP - TT | 1/3 | 0.5 | 25 | 34 | 29 |
| PRP-TT | 1/2 | 0.6 | 18 | 8 | 40 |
| PRP-TT | 1/1 | 0.3 | 5 | 19 | 38 |

**TABLE 2**

| **Vaccine** | **PS/TT** | **∝-PRP (γ/ml)** | | | |
|---|---|---|---|---|---|
| | | **Alone** | | **+DTPa** | |
| | | **d24** | **d38** | **d24** | **d38** |
| Nacl | - | 0.002 | 0.01 | 0.002 | 0.01 |
| Hib001 A44 (control) | 1/2.5 | 0.08 | 51.5 | 0.63 | 40.3 |
| Dhib015 | 1/3 | 0.88 | 64.1 | 0.47 | 19 |
| Dhib026 | 1/1 | 0.07 | 9.1 | 1.27 | 66.7 |

**TABLE 3**

| **Vaccine** | **PS/TT** | **∝-PRP Abs(γ/ml)** | | | |
|---|---|---|---|---|---|
| | | **Alone** | | **+ DTPa** | |
| | | **d24** | **d38** | **d24** | **d38** |
| Nacl | - | 0.002 | 0.01 | 0.002 | 0.01 |
| Hib001 A44 (control) | 1/2.5 | 0.05 | 7.0 | 0.09 | 14.4 |
| Dhib015 | 1/3 | 0.07 | 17.7 | 0.12 | 14.7 |
| Dhib026 | 1/1 | 0.02 | 0.45 | 0.17 | 51.6 |

**TABLE 4**

| **Vaccine** | **Ab response after 3 doses (d 42)** | | | | | | |
|---|---|---|---|---|---|---|---|
| | **∝-PRP** | **∝-D** | **∝-T** | **∝-PT** | **∝-FHA** | **∝-69k** | **∝-HBS** |
| None (NaCl) | <0.1 | <3 | <2 | <8 | <4 | <2 | <2 |
| DTPa + Hib | 3.4 | 2847 | 6976 | 1518 | 2119- | 127 | - |
| (Control PS/TT-1/2.5) | | | | | N | | |
| | | | | | S | | - |
| + Hib (low ratio) (PS/TT ~ 1/1) | 20.9 | 2847 | 9579 | 2144 | 3551- | 35 | |
| DTPaHB + Hib | 2.8 | 2001 | 3622 | 1603 | 1123- | 18 | 3622 |
| (PS/TT-1/2.5) | | | | | N | | |
| | | | | | S | | 6674 |
| + Hib (low ratio) (PS/TT - 1/1) | 11.4 | 1874 | 6674 | 1402 | 3035- | 12 | |

## Claims

1. A combined vaccine comprising:
a) a polysaccharide antigen derived from Haemophilus influenzae type B, Steptococcus pneumoniae, or Neisseria meningitidis A, B or C, conjugated to a carrier protein; and
b) a DTPa vaccine comprising diphtheria toxoid, tetanus toxoid and an acellular pertussis component;
**characterised in that** the ratio of polysaccharide to carrier protein in the conjugate is from 1:0.3 to 1:2 (w/w) and **in that** the carrier protein is tetanus toxoid which is also present as free antigen in the DTPa vaccine.

2. A combined vaccine as claimed in claim 1 wherein the ratio of polysaccharide: carrier protein in the conjugate is from 1:0.5 to 1:1.5 (w/w).

3. A combined vaccine as claimed in claim 1 further comprising an antigen derived from hepatitis B virus.

4. A combined vaccine as claimed in any one of claims 1 or 3 further comprising an antigen against hepatitis A virus.

5. A combined vaccine as claimed in any one of claims 1, 3 or 4 further comprising an antigen providing immunity against polio.

6. A combined vaccine according to any one of claims 1, 3, 4 or 5 additionally comprising a suitable adjuvant.

7. A vaccine as claimed in any of the previous claims, for use in medicine.

## Patentansprüche

1. Kombinationsimpfstoff, umfassend:
(a) ein Polysaccharidantigen, das von Haemophilus influenzae-Typ B, Streptococcus pneumoniae oder Neisseria meningitidis A, B oder C stammt, konjugiert mit einem Trägerprotein; und
(b) einen DTPa-Impfstoff umfassend Diphtherietoxoid, Tetanustoxoid und einen azellulären Pertussisbestandteil;
**dadurch gekennzeichnet, dass** das Verhältnis von Polysaccharid zu Trägerprotein im Konjugat 1:0,3 bis 1:2 (Gew./Gew.) beträgt und dass das Trägerprotein das Tetanustoxoid ist, das auch als freies Antigen im DTPa-Impfstoff vorhanden ist.

2. Kombinationsimpfstoff nach Anspruch 1, wobei das Verhältnis von Polysaccharid zu Trägerprotein im Konjugat 1:0,5 bis 1:1,5 (Gew./Gew.) beträgt.

3. Kombinationsimpfstoff nach Anspruch 1, der ferner ein Antigen umfaßt, das vom Hepatitis B-Virus stammt.

4. Kombinationsimpfstoff nach einem der Ansprüche 1 oder 3, der ferner ein Antigen gegen das Hepatitis A-Virus umfasst.

5. Kombinationsimpfstoff nach einem der Ansprüche 1, 3 oder 4, der ferner ein Antigen umfaßt, das Immunität gegen Polio hervorruft.

6. Kombinationsimpfstoff nach einem der Ansprüche 1, 3, 4 oder 5, der zusätzlich ein geeignetes Adjuvans umfaßt.

7. Impfstoff nach einem der vorangehenden Ansprüche zur Verwendung in der Medizin.

## Revendications

1. Vaccin combiné comprenant : (a) un antigène polysaccharidique dérivé d'Haemophilus Influenzae de type B, de Streptococcus Pneumonia ou de Neisseria meningitis A, B ou C, conjugué à une protéine porteuse ; et (b) un vaccin DTPa comprenant une anatoxine de diphtérie, une anatoxine du tétanos et un composant acellulaire de periussis ; **caractérisé en ce que** le rapport entre le polysaccharide et la protéine porteuse dans le conjugué est de 1:0,3 à 1:2 (P/P) et **en ce que** la protéine porteuse est une anatoxine du tétanos, qui est présente aussi en tant qu'antigène libre dans le vaccin DTPa.

2. Vaccin combiné selon la revendication 1 dans lequel le rapport entre le polysaccharide et la protéine porteuse dans le conjugué est compris entre 1:0,5 et 1:1,5 (P/P).

3. Vaccin combiné selon la revendication 1 comprenant en outre un antigène dérivé du virus de l'hépatite B.

4. Vaccin combiné selon la revendication 1 ou 3 comprenant en outre un antigène contre le virus de l'hépatite A.

5. Vaccin combiné selon l'une quelconque des revendications 1, 3 ou 4 comprenant en outre un antigène apte à induire une immunité contre la polio.

6. Vaccin combiné selon l'une quelconque des revendications 1, 3, 4 ou 5 comprenant de plus un adjuvant approprié.

7. Vaccin selon l'une quelconque des revendications précédentes pour une utilisation en médecine.
